(19) Europäisches Patentamt
European Patent Office
Office européen des brevets

(11) Publication number: **0 001 005**
**A1**

(12) **EUROPEAN PATENT APPLICATION**

(21) Application number: **78300299.1**

(22) Date of filing: **18.08.78**

(51) Int. Cl.²: **C 07 D 233/10**, C 07 D 233/14

(30) Priority: **18.08.77 GB 34810/77**

(43) Date of publication of application: **07.03.79**
**Bulletin 79/5**

(84) Designated Contracting States: **BE CH DE FR GB LU NL SE**

(71) Applicant: **Albright & Wilson Limited, Albright & Wilson House Hagley Road West, Oldbury Warley West Midlands (GB)**

(72) Inventor: **Phillips, Bringley Morris, Springfield Villas Egremont Road, Hensingham Whitehaven Cumbria (GB)**
Inventor: **Lamble, Alan James, 7 Lansdowne Grove, Whitehaven Cumbria (GB)**

(74) Representative: **Savidge, Roger Gordon Madgwick et al, c/o Albright & Wilson Limited 1 Knightsbridge Green, London SW1X 7QD (GB)**

(54) **Manufacture of imidazolines.**

(57) The reaction between a fatty acid RCOOH and a diamine $H_2NCH_2CH_2NHR_1$ wherein R represents an aliphatic group comprising from 8 to 22 carbon atoms and $R_1$ represents a substituted or unsubstituted alkyl or alkenyl group comprising from 1 to 4 carbon atoms so as to produce an imidazoline is carried out using a moderate initial excess of diamine and when necessary reducing the pressure logarithmically with respect to time until the reaction is completed.

The formation of undesirable diamide impurities is avoided while using economic quantities of diamine and non-specialised equipment. The processes can be carried out without any monitoring of the progress of the reaction by skilled personnel being required and if through error progress is unsatisfactory a single readjustment can serve to carry the reaction to completion.

EP 0 001 005 A1

This invention relates to novel methods and apparatus for carrying out and controlling the condensation reaction between a fatty acid and an ethylene diamine to produce an imidazoline compound.

The imidazolines in question are those having the general formula:

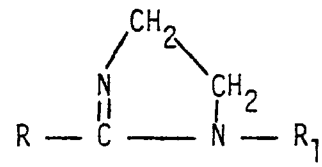

wherein R represents an aliphatic group which comprises from 8 to 22 carbon atoms per molecule and $R_1$ represents a substituted or unsubstituted alkyl or alkenyl group comprising from 1 to 4 carbon atoms.

The preparation of such compounds by the condensation of a fatty acid RCOOH with a diamine $H_2NCH_2CH_2NHR_1$ has been described in United States Patent 2,267,965. The reaction proceeds substantially according to the equation:

$$RCOOH + H_2NCH_2CH_2NHR_1 \rightleftharpoons RCONHCH_2CH_2NHR_1 + H_2O$$

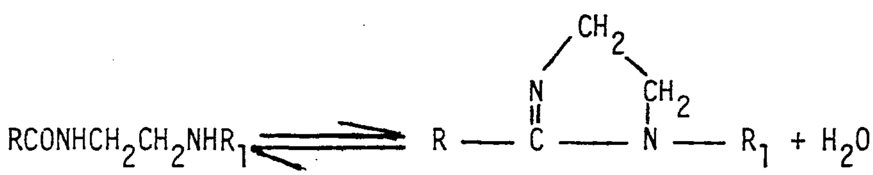

A problem in carrying out this reaction is that the diamine is relatively volatile under the conditions used and tends to distil out of the reaction system with the water of reaction which is necessarily removed from the system. This is a particular problem since as the proportion of amine present drops the intermediate amido-amine increasingly tends to react with a further molecule of acid to form a diamide. Where the imidazoline product is subsequently converted into a derivative which is useful as a surface active agent such as those described in United States Patents 2,528,378, 2,773,068 and 3,738,996 and incorporated into an aqueous composition the diamide impurity separates from the solution as a white, cloudy material which detracts from the ideal sparkling clear nature of the product.

In British Patent 1,187,131 it is suggested that in order to minimise the formation of diamide the reaction should be carried substantially to completion in the presence of at least an 8% molar excess of the diamine. This is achieved by the repeated addition of small quantities of amine to the reactor.

A variety of techniques can be adopted in order to maintain the excess of diamine. The reaction can be carried out using a very large

initial excess of diamine so that the required excess quantities are present even at the end of the reaction; provision can be made to supply fresh diamine to the reaction vessel as the reaction proceeds or an extremely efficient fractionating system can be fitted so as to prevent the escape of any significant quantity of diamine. In a commercial scale manufacturing operation the first method is unattractive for economic reasons whilst the second and third require that the progress of the reaction be monitored extremely carefully. During the reaction the pressure must be reduced in order to remove the water of reaction. If this is done too quickly the boil up rate of the liquid increases in an uncontrollable manner and if it is done too slowly distilliation ceases and the reaction time is extended. Such techniques pose problems of plant control.

We have now discovered a method for controlling the reaction between a fatty acid and a diamine so as to produce a product having a low content of diamide which requires only a moderate initial excess of diamine and avoids the necessity of replenishing the diamine during the course of the reaction. Our invention provides a process for the preparation of a compound having the formula:-

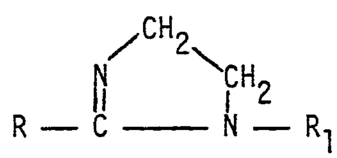

wherein R and $R_1$ are as hereinbefore defined which comprises heating a fatty acid RCOOH in the presence of an excess of a diamine $H_2NCH_2CH_2NR_1$ until at least 1.8 moles of water per mole of acid have been evolved, the pressure being reduced as necessary from its initial value to a final value in the range 5 to 50 mm of mercury in a substantially logarithmic manner with respect to time.

Surprisingly this pattern of the variation of pressure with time controls the vapour temperature so as to minimise excessive boil up and hence loss of diamine whilst maintaining a steady reaction rate. The initial excess of diamine required to ensure than an excess is present throughout the reaction can be reduced from the very high levels required in previous processes which were not be monitored to a level of from 12 to 30% (expressed as moles of diamine to moles of acid) without producing a product comprising too high a proportion of diamine. Furthermore the reaction can be carried out by unskilled personnel or more preferably by employing automatic equipment programmed to reduce the pressure in a logarithmic manner.

The reaction is carried out at a temperature of greater than 150°C but less than boiling point of the amine under the conditions employed. Preferably the temperature in the pot is maintained in the range 170° to 220°C, most preferably 180° to 200°C.

The novel procedures are conveniently carried out by charging approximate quantities of acid and diamine to a closed vessel equipped with external or internal heating means; a fractionating condenser and a source of vacuum. The temperature of the reactants is raised to the desired level sufficiently slowly so as to minimise frothing. During or after but preferably before this operation the vessel is evacuated so as to reduce the pressure to a preselected initial level which ensures adequate evaporation of water so that the reaction proceeds at a satisfactory rate whilst minimising the evaporation of amine. A suitable level can be ascertained by experiment using a particular reaction system but we have found the following correlation to represent convenient temperature/pressure setting:

| Temperature $^{o}C$ | Pressure mm Hg |
|---|---|
| 200 | 200 mm |
| 175 | 150 mm |
| 150 | 100 mm |

The reaction will commence at higher pressures but will not proceed toward completion with any great rapidity. However if the indicated temperatures and pressures are employed and maintained up to 50% of the reaction may take place without any further reduction in pressure being required. When the reaction slows which is indicated by a rise in vapour temperature, the pressure is reduced logarithmically with respect to time. The time necessary to complete the reaction to the required degree is also determined by experiment. In general, when operating in our preferred temperature ranges, total reaction times of from 12 to 24 hours may be required, the pressure being reduced over a period of from 6 to 18 hours of that time.

For a reaction carried out using a particular fractionating system the pressure at any given time t can be calculated from the equation

$$Log_x P = Log_x Po-(Log_x 10).A.t$$

where $P_o$ is the pressure when time t is 0, x is the base upon which the logarithm is expressed and A represents a constant.

When the pressure P is expressed as millimetres of mercury (absolute) and the time t in hours we have found that values of A from 0.03 to 0.4 lead to the formation of products containing acceptably low quantities of diamide in acceptable short periods of time. Preferably A takes a value of from 0.06 to 0.25.

We have found that variations in either $log_x P$ or of the constant A of $\pm$ 10% from the ideal value for a particular system can be tolerated without leading to undesirably high rates of boil up or prolonging the reaction unduly. In practice some deviation of $log_x P$ is enevitable whilst the tolerence allowed in selecting a value of A is useful in avoiding tedious evaluation of the ideal value.

Where the rate of boil up during the reaction becomes too high or too slow (thereby prolonging the reaction) the rate of pressure reduction may be adjusted accordingly. Thus in effect in such a circumstance the rate of reduction is recalculated on the basis of a

fresh set of $P_0$ and $t_0$ values corresponding to the point reached during the reaction. Such techniques are less preferred since they require that the progress of the reaction should be monitored. Normally in a particular system no more than two and certainly no more than five such adjustments should be necessary. In fact it may well be preferable to allow the rate of boil to exceed the optimum for a short time since any deficiencies in the product of that particular reaction can be remedied by blending it with the product of other more successful reactions, rather than to monitor the progress of the reaction and the pressure on frequent occasions.

The preferred fatty acids for use in the processes of our invention are those in which R represents an alkyl, alkenyl or cycloalkyl group comprising from 8 to 22 carbon atoms. Conveniently such acids are obtained from natural sources and comprise mixtures of acids in which the nature of the group R varies. Suitable acids include caproic, heptylic, capryllic, undecylic, lauric, palmitic, stearic, isostearic,ethylhexanonic , behenic, arachic, cerotic, oleic, erucic, linoleic, linolenic and ricinoleic acids.

Natural mixtures useful include the so-called coconut acids, palm oil acids and tallow acids.

Preferably such acids comprise an average of, from 10 to 18 most preferably 12 to 14 carbon atoms per molecule.

The diamine reactants preferred for present use are those wherein $R_1$ represents a alkyl, hydroxy alkyl or amino alkyl group having from 1 to 4 carbon atoms. Preferably $R_1$ represents a hydroxy alkyl group having from 2 to 4 carbon atoms. Most preferably $R_1$ represents a hydroxy ethyl group $CH_2CH_2OH$. Thus the most preferred diamine from which the imidazoline is derived is N-(2-aminoethyl)-ethanolamine.

The invention is illustrated by following example:-

EXAMPLE I :

A stirred jacketed reactor was charged with 68 kg lauric acid and 46.1 kg aminoethylethanolamine.

The reactants were heated to 140°C under 200mm Hg abs. pressure. The temperature was then raised to 190°C at a rate of 10°/hr, maintaining the pressure at 200 mm Hg Abs.

The pressure was then reduced logarithmically, maintaining the temperature of the reactants at 190°C - 195°C.

The conditions for the reaction are shown in the table below.

| Time (hrs) | Temp Reactants. | Pressure (mm Hg Abs.) | %Theoretical water removed | %Mole excess amine in reactor |
|---|---|---|---|---|
| 0 | 140 | 200 | 0 | 30 |
| 1 | 148 | 200 | 9.9 | 29.3 |
| 2 | 160 | 200 | 29.8 | 28.8 |
| 3 | 170 | 200 | 42.7 | 28.4 |
| 4 | 180 | 200 | 50.1 | 28.2 |
| 5 | 190 | 200 | 56.1 | 28.0 |
| 6 | 193 | 162 | 60.9 | 27.8 |
| 7 | 195 | 139 | 64.4 | 27.6 |
| 8 | 194 | 110 | 66.4 | 27.6 |
| 9 | 196 | 90 | 70.4 | 27.5 |
| 10 | 196 | 73 | 73.3 | 27.3 |
| 11 | 196 | 60 | 75.2 | 27.2 |
| 12 | 194 | 48 | 77.4 | 27.0 |
| 13 | 194 | 39.5 | 78.9 | 26.8 |
| 14 | 190 | 32 | | |
| 15 | 190 | 27 | 80.8 | 26.5 |
| 16 | 190 | 22 | 82.5 | 26.0 |
| 17 | 190 | 18 | 84.0 | 25.1 |
| 18 | 188 | 14 | 85.6 | 24.0 |
| 19 | 186 | 12 | 86.5 | 22.9 |
| 20 | 187 | 10 | 88.7 | 17.8 |

The pressure was then reduced more rapidly to 3mm Hg abs. to remove the remaining water of reaction and excess amine.

The product was cooled to 60°C under vacuum, and an analysis was found to contain 1.9% diamide. On the basis of the calculation outlined above the constant A had a value of 0.087.

EXAMPLE II

A stirred reactor was charged with 644kg coconut fatty acid and 395kg aminoethylethanolamine.

The reactants were heated to 140°C under 200mm Hg pressure. The temperature was then raised to 182°C at a rate of 10°/hr, maintaining the pressure at 200mm Hg abs.

The pressure was then reduced logarithmically maintaining the temperature of the reactants at 180-185°C.

The conditions for the reaction are shown below:

| Time (hrs) | Temp °C Reactants | Pressure (mm Abs) | % Total water removed | % Mole excess amine in reactor * |
|---|---|---|---|---|
| 1 | 147 | 205 | 44.1 | 18.4 |
| 3 | 162 | 205 | 58.6 | 18.2 |
| 5 | 177 | 202 | 68.0 | 18.0 |
| 6 | 182 | 201 | 71.1 | 18.0 |
| 8 | 183 | 130 | 78.7 | 17.9 |
| 10 | 182 | 79 | 86.3 | 17.7 |
| 12 | 182 | 46 | 94.4 | 17.5 |
| 14½ | 181 | 31 | 98.1 | 15.8 |
| 16 | 181 | 20 | 99.0 | 11.5 |

The pressure was then reduced to 3mm Hg abs. to remove the excess amine.

* calculated on original charge of acid.

The product was cooled to 60°C under vaccum, and on analysis was found to contain 0.6% diamide.

In this example the value of the constant A was 0.103.

What we claim is:-

1. A process for the preparation of a compound having the formula

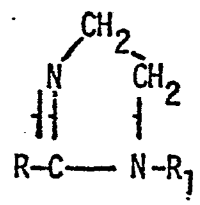

wherein R represents an aliphatic group which comprises from 8 to 22 carbon atoms and $R_1$ represents a substitutal or unsubstituted alkyl or alkenyl group comprising from 1 to 4 carbon atoms; which comprises heating a fatty acid RCOOH in the presence of an excess of a diamine $H_2NCH_2CH_2NHR_1$ until at least 1.8 moles of water per mole of acid have been evolved characterised in that the pressure is reduced as necessary from its initial value to a final value in the range 5 to 50mm of mercury in a substantially logarithmic manner with respect to time.

2. A process according to claim 1 characterised in that $R_1$ represents a hydroxy alkyl group having from 2 to 4 carbon atoms.

3. A process according to claim 2 characterised in that $R_1$ represents a hydroxy ethyl group.

4. A process according to any of the preceding claims characterised in that the group R comprises from 10 to 18 carbon atoms.

5. A process according to claim 4 characterised in that the group R comprises from 12 to 14 carbon atoms.

6. A process according to any of the preceding claims characterised in that the amine is initially present in an excess of from 12 to 30% (expressed as moles of diamine to moles of acid).

7. A process according to any of the preceding claims characterised in that the reaction is carried at a temperature of from 170 to 220°C.

8. A process according to claim 7 characterised in that the reaction is carried out at a temperature of from 180 to 200°C.

9. A process according to any of the preceding claims characterised in that the pressure P at a given time t can be calculated from the equation.

$$\log_x P = \log_x P_0 - (\log_x 10)A.t$$

where $P_0$ is the pressure at time t = 0, x is the base upon which the logarithm is expressed and A is a constant.

10. A process according to claim 9 characterised in that where P is expressed in millimetres of mercury (absolute) and t is expressed in hours A has a value of from 0.03 to 0.4.

11. A process according to claim 10 characterised in that A has a value of from 0.06 to 0.25.

European Patent Office

# EUROPEAN SEARCH REPORT

0001005

Application number
EP 78 30 0299

## DOCUMENTS CONSIDERED TO BE RELEVANT

| Category | Citation of document with indication, where appropriate, of relevant passages | Relevant to claim |
|---|---|---|
| A | DE - A - 1 795 359 (MANNHEIMER)<br>* Pages 10 to 17 *<br>-- | 1,4-6 |
| DA | GB - A - 1 187 131 (MANNHEIMER)<br>(Cited in the application)<br>* Pages 1 to 5 *<br>-- | 1-3,6 |
| DA | US - A - 2 528 378 (MANNHEIMER)<br>* Columns 1 to 4 *<br>---- | 1 |

CLASSIFICATION OF THE APPLICATION (Int. Cl.[2])

C 07 D 233/10
C 07 D 233/14

TECHNICAL FIELDS SEARCHED (Int.Cl.[2])

C 07 D 233/10
C 07 D 233/14

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| The Hague | 13-11-1978 | DE BUYSER |

EPO Form 1503.1 06.78